Europäisches Patentamt

European Patent Office

Office européen des brevets

(19)

(11) Numéro de publication : **0 496 173 B1**

(12) **FASCICULE DE BREVET EUROPEEN**

(45) Date de publication du fascicule du brevet :
02.03.94 Bulletin 94/09

(51) Int. Cl.$^5$ : **A61K 7/48,** A61K 35/78

(21) Numéro de dépôt : **91400899.0**

(22) Date de dépôt : **03.04.91**

(54) **Extraits de Noix d'Alep, leur préparation et leurs applications.**

(30) Priorité : **22.01.91 FR 9100696**

(43) Date de publication de la demande :
**29.07.92 Bulletin 92/31**

(45) Mention de la délivrance du brevet :
**02.03.94 Bulletin 94/09**

(84) Etats contractants désignés :
**AT BE CH DE DK ES FR GB GR IT LI LU NL SE**

(56) Documents cités :
BEZANGER et al.: "Plantes médicinales des régions tempérées", 1980, pages 74-75: "Quercus lusitanica lamk.", Maloine S.A., Paris, FR
STN SERVEUR DE BASES DE DONNEES, Karlsruhe, DE, FICHIER CHEMICAL ABSTRACTS, vol. 87, no. 3, abrégé no. 19007a, Columbus, Ohio, US; M. IKRAM et al.: "Constituents of Quercus infectoria", & PLANTA MED., 31(3), 286-7
STN SERVEUR DE BASES DE DONNEES, Karlsruhe, DE, FICHIER CHEMICAL ABSTRACTS, vol. 106, no. 5, abrégé no. 31339d, Columbus, Ohio, US; M.H. SALAGOITY-AUGUSTE et al.: "Preliminary investigation for the differentiation of enological tannins according to botanical origin: determination of gallic acid and its derivatives", & AM. J. ENOL. VITIC., 37(4), 301-3

(56) Documents cités :
PATENT ABSTRACTS OF JAPAN, vol. 96, (C-412), 1 novembre 1986; & JP-A-61 246 109 (RIYUUHOUDOU SEIYAKU K.K.)
STN SERVEUR DE BASES DE DONNEES, Karlsruhe, DE, FICHIER CHEMICAL ABSTRACTS, vol. 99, no. 11, abrégé no. 85071r, Columbus, Ohio, US; M. NISHIZAWA et al.: "Tannins and related compounds. Part 9. Isolation and characterization of polygalloylglucoses from Turkish galls (Quercus infectoria)", & J. CHEM. SOC., PERKIN TRANS. 1, (5), 961-5

(73) Titulaire : **SYNTHELABO**
**22, Avenue Galilée**
**F-92350 Le Plessis Robinson (FR)**

(72) Inventeur : **Fabre, Bernard**
**4, rue du Pressoir**
**F-37270 Saint-Martin-le-Beau (FR)**
Inventeur : **Potier, Anne**
**39-41, rue Emile-Zola Appt 10**
**F-37100 Tours (FR)**
Inventeur : **Fontanel, Didier**
**16, rue de la Gatine**
**F-37390 Notre-Dame-D'Oe (FR)**
Inventeur : **Duvnjak, Philippe**
**9, rue de Bois Billières**
**F-37230 Fondettes (FR)**

(74) Mandataire : **Thouret-Lemaitre, Elisabeth et al**
**SYNTHELABO, Service Brevets, B.P. 72**
**F-92352 Le Plessis-Robinson Cédex (FR)**

## Description

La présente invention a pour objet des extraits de Noix de Galle appelée également Noix d'Alep, ayant des propriétés de filtres protecteurs des U.V.B. et un rôle préventif contre les effets nocifs des radicaux libres (responsables du vieillissement cutané), leur obtention et leurs applications.

L'apparition des Noix de Galle ou Noix d'Alep (Quercus infectoria Oliv.) familles des fagacées est provoquée par la ponte d'un hyménoptère (Cynips Gallae tinctoria Oliv.) dans les jeunes bourgeons de chêne.

Les chênes à Noix de Galle sont répandus principalement en Asie Mineure (Iran, Syrie, Turquie). On en trouve également en Grèce.

Les principaux composants chimiques de la Noix d'Alep sont les suivants :
- Tanins hydrolysables (50 à 70 %)
- Acide gallique et ses esters
- Acide ellagique
- Acide syringique
- Glucides dont amidon
- Sitostérol
- Oléate de méthyle, bétulate de méthyle.

Des extraits de Noix d'Alep sont décrits dans le document "BEZANGER et al.: Plantes médicinales des régions tempérées, 1980, pages 74-75" ou dans le document "Patent Abstracts of Japan, vol. 96, (C-412), 1/11/1986.

Les extraits de l'invention sont obtenus selon le procédé décrit ci-dessous :

selon l'invention, on soumet la Noix d'Alep, sèche ou fraîche, broyée ou pulvérisée à une extraction par un solvant tel que l'eau, un alcanol en $C_1$-$C_4$, l'acétone ou le propylène glycol, ou un mélange de ces solvants. Par exemple, on peut utiliser un mélange alcanol $C_1$-$C_4$/eau ou un mélange acétone/eau en proportions de 96/4 à 30/70 en volume. On peut également utiliser un mélange propylèneglycol/eau dans des proportions de 100/10 à 40/60 en volume.

La quantité de solvant d'extraction utilisée est égale à 4 à 20 fois le poids de Noix d'Alep.

On effectue l'extraction éventuellement sous agitation, à une température située entre 10°C et l'ébullition du solvant. La durée de l'extraction est de 2 à 80 heures.

On concentre éventuellement les solutions extractives. Les concentrats obtenus sont éventuellement séchés, soit en étuve sous vide, soit par nébulisation, soit par lyophilisation, soit par séchage à l'aide d'un sécheur-réacteur cylindrique.

Les extraits de Noix d'Alep selon l'invention sont caractérisés par la présence d'acide gallique, d'acide ellagique et de tanins hydrolysables. Ils ne contiennent pas d'acide protocatéchique. Dans les extraits de Noix d'Alep de l'invention, la teneur en acide gallique varie de 0,5 à 7 % en poids, et la teneur en tanins varie de 65 à 85 % en poids par rapport à la poudre pour un extrait sec et par rapport au résidu sec pour un extrait liquide ou mou.

Ces différents constituants ont été mis en évidence par les tests décrits ci-après. Leur dosage est effectué de la manière indiquée ci-dessous :

l'acide gallique, les tanins et l'acide ellagique peuvent être mis en évidence par des réactions colorées : on met en présence une solution hydroéthanolique d'extrait de Noix d'Alep de l'invention et une solution de cyanure de potassium. Une coloration rouge-orangé apparait. Cette coloration caractéristique de l'acide gallique, est suivie d'un précipité jaune caractéristique des tanins.

D'autre part, une solution hydroéthanolique (à 60 % d'éthanol en volume) d'extrait de Noix d'Alep de l'invention et d'hypochlorite de sodium prend une coloration orange caractéristique de l'acide ellagique.

On peut identifier les acides gallique et ellagique par chromatographie sur couche mince de gel de silice. On utilise une phase mobile constituée d'un mélange de benzène, de méthanol et d'acide acétique dans les proportions 45-8-3. Après migration, on pulvérise une solution hydroéthanolique à 2 % en volume de chlorure ferrique, ce qui révèle la présence d'acide gallique et d'acide ellagique.

On peut aussi identifier les tanins lors de l'expérience suivante :

une solution hydroéthanolique d'extrait de Noix d'Alep obtenu selon l'invention, à une concentration de 1 mg d'extrait de Noix d'Alep par millilitre de solution, et contenant 10 % en volume de chlorure ferrique, précipite. Ce précipité, de couleur bleu-noir, est caractéristique des tanins galliques.

On dose l'acide gallique par chromatographie liquide haute performance. La colonne utilisée est de type silice greffée phase inverse $C_{18}$. La phase mobile est composée d'un mélange en proportions variables de méthanol et d'eau acidifiée par 2,5 % en volume d'acide acétique. Le débit est de 1 ml/mn ; la détection s'effectue à 254 nm. Les teneurs en acide gallique varient de 1,5 à 7 % en poids par rapport au résidu sec pour un extrait liquide ou mou, suivant la nature des solvants d'extraction et la méthode d'extraction.

On dose les tanins selon la technique d'adsorption sur poudre de peau. Cette méthode de dosage est décrite dans la Pharmacopée française 9e et 10e éditions, pour le dosage des tanins dans les plantes. La teneur en tanins hydrolysables de l'extrait de Noix d'Alep de l'invention varie de 65 à 85 % en poids par rapport à la poudre pour un extrait sec et par rapport au résidu sec pour un extrait liquide ou mou, suivant la nature des solvants d'extraction et la méthode d'extraction.

L'absence d'acide protocatéchique est mise en évidence par l'absence de réaction d'une solution aqueuse de l'extrait de Noix d'Alep de l'invention en présence d'acétate de cuivre.

Les exemples suivants illustrent l'invention :

Exemple 1.

On introduit 5 kg de noix d'Alep broyée dans un percolateur. On ajoute 38 kg d'une solution hydroéthanolique à 50 % en volume d'éthanol. On laisse macérer le mélange pendant 10 heures. Puis on soutire les liqueurs d'extraction pendant 48 heures. On concentre les liqueurs obtenues dans un évaporateur sous pression réduite et à 50°C. On sèche alors le concentrat par nébulisation, puis on homogénéise le produit sec. Celui-ci se présente sous la forme d'une poudre de couleur beige. Les réactions colorées des tanins et des acides gallique et ellagique sont positives. On identifie également les acides gallique et ellagique par chromatographie sur couche mince.

On dose les tanins suivant la méthode décrite précédemment : leur teneur est de 75 % en poids par rapport à la poudre. On dose l'acide gallique suivant la méthode décrite précédemment : sa teneur varie de 1,5 à 3,0 % en poids par rapport à la poudre.

Exemple 2.

On introduit 10 g de Noix d'Alep pulvérisée dans un réacteur chauffant. On ajoute 100 g d'une solution hydroéthanolique à 96 % en volume d'éthanol. On porte à ébullition pendant 2 heures, puis on filtre sous vide. On concentre le filtrat à sec sous pression réduite et à 60°C. Puis on broie et on homogénéise le produit sec. Celui-ci se présente sous forme d'une poudre fine de couleur beige. Les réactions colorées des tanins et des acides gallique et ellagique sont positives. On identifie également les acides gallique et ellagique par chromatographie sur couche mince.

On dose les tanins suivant la méthode décrite précédemment, leur teneur est de 80 % en poids par rapport à la poudre. On dose l'acide gallique suivant la méthode décrite précédemment : sa teneur varie de 1,5 à 3,0 % en poids par rapport à la poudre.

Exemple 3.

On introduit 10 g de Noix d'Alep pulvérisée dans un erlenmeyer. On y ajoute 100 ml d'une solution hydroacétonique à 40 % en volume d'acétone. On agite pendant 3 heures à la température de 20°C. On filtre. On concentre le filtrat à sec, sous pression réduite, à 50°C, puis on broie et on homogénéise le produit sec. Il se présente sous la forme d'une poudre fine de couleur beige. Les réactions colorées des tanins et des acides gallique et ellagique sont positives. On identifie également les acides gallique et ellagique par chromatographie sur couche mince. On dose les tanins suivant la méthode décrite précédemment : leur teneur est de 80 % en poids par rapport à la poudre. On dose l'acide gallique suivant la méthode décrite précédemment : sa teneur varie de 5 à 7 % en poids par rapport à la poudre.

Exemple 4.

On introduit 10 g de Noix d'Alep pulvérisée dans un erlenmeyer. On y ajoute 100 g d'eau. On chauffe pendant 2 heures à 50°C. On filtre, puis on concentre le filtrat sous pression réduite à 75°C jusqu'à consistance pâteuse. La pâte présente une couleur brune. Les réactions colorées des tanins et des acides gallique et ellagique sont positives. On identifie également les acides gallique et ellagique par chromatograhie sur couche mince. On dose les tanins : leur teneur est de 75 % en poids par rapport au résidu sec de l'extrait. On dose l'acide gallique suivant la méthode décrite précédemment : sa teneur varie de 3,5 à 5,5 % en poids par rapport au résidu sec de l'extrait.

Exemple 5.

On introduit 20 g de Noix d'Alep broyée dans un percolateur. On ajoute 200 g d'une solution hydroétha-

3

nolique à 50 % en volume d'éthanol. On laisse macérer le mélange pendant 12 heures. Puis on soutire les liqueurs d'épuisement pendant 36 heures. Les réactions colorées des tanins et des acides gallique et ellagique sont positives.

On identifie également les acides gallique et ellagique par chromatographie sur couche mince. On dose les tanins suivant la méthode décrite précédemment : leur teneur est de 80 % en poids par rapport au résidu sec de l'extrait. On dose l'acide gallique suivant la méthode décrite précédemment : sa teneur varie de 1,5 à 3,0 % en poids par rapport au résidu sec de l'extrait.

On a étudié l'activité des extraits de Noix d'Alep obtenus selon l'invention.

On met ainsi en évidence deux propriétés : l'activité filtre U.V.B. et l'activité anti-radicalaire.

On détermine l'activité filtre U.V.B. des extraits obtenus selon l'invention à partir des observations suivantes :

les extraits de Noix d'Alep ont la particularité d'absorber les rayonnements U.V. et plus particulièrement les U.V.B..

La peau, lors d'un excès d'irradiation lumineuse est agressée par les U.V.A. et les U.V.B..

Les U.V.A. par un mécanisme complexe, provoquent l'apparition de radicaux libres toxiques qui sont responsables d'un vieillissement prématuré de la peau. La toxicité des U.V.B. peut être très large. Elle peut aller d' une simple inflammation des tissus cutanés (coup de soleil) à l'inactivation de processus enzymatiques essentiels et certains cancers de la peau.

La zone d'absorption des U.V. d'une solution hydroéthanolique d'un extrait de Noix d'Alep se situe entre 200 et 350 nm avec 2 maxima, le premier entre 200 et 240 nm, le second entre 240 et 350 nm. Ce dernier se situe dans la zone des U.V.B..

Les extraits de Noix d'Alep obtenus selon l'invention permettent donc une protection contre les U.V.B. et leurs effets toxiques.

On détermine l'activité anti-radicalaire des extraits de Noix d'Alep obtenus selon l'invention à partir des observations suivantes.

Les radicaux libres OH· (radical hydroxyle) et $O_2^{\cdot-}$ (anion superoxyde) sont des molécules très instables qui cherchent à apparier leur électron célibataire. De telles molécules sont produites normalement en quantité très faible au cours de la vie cellulaire. Elles sont principalement formées lors de réactions d'oxydation physiologiques. Des enzymes comme la superoxyde dismutase ou la catalase, sont capables de les capter ou de les détruire. Mais elles peuvent être présentes à des concentrations anormalement élevées suite à des phénomènes comme une déficience enzymatique ou sous l'action des rayons U.V. lors d'une exposition au soleil. La peau est particulièrement sujette à ce mode de formation. Les radicaux libres, hautement réactifs peuvent alors réagir avec certains constituants clés de la cellule et les altérer.

Les constituants cellulaires qui représentent les cibles privilégiées des radicaux libres sont les suivants :

- les acides gras polyinsaturés entrant dans la constitution des phospholipides des membranes cellulaires,
- les protéines enzymatiques, les protéines de structures (collagène, élastine...), les acides nucléiques.

L'altération des acides gras insaturés peut engendrer la formation de lipopéroxydes cytotoxiques avec désorganisation des membranes dans l'épiderme. Les conséquences sont la déshydratation, des phénomènes inflammatoires, etc... Une altération des acides aminés et nucléiques peut provoquer des dommages d'ordre biochimique irréparables, qui ne sont pas sans influence sur le vieillissement de la peau.

Afin d'éviter les risques causés par les radicaux libres, deux possibilités existent :

- éviter la formation des radicaux libres,
- éliminer les radicaux libres en les "neutralisant".

On a pu évaluer l'activité anti-radicalaire des extraits de Noix d'Alep selon l'invention par leur action sur un radical libre, le diphénylpicrylhydrazylhydrate absorbant dans le violet à 513 nm. (Lamaison J.L., Petitjean-Freytet C., Carnat P., Carnat A., 1988, Plantes Medicinales et Phytothérapie, 22,(4), p. 231-234). Un composé anti-radicalaire, piégeur de radicaux libres, entraîne une inhibition de l'apparition de colororation du radical libre témoin, donc une diminution de la densité optique de ce radical : l'activité anti-radicalaire d'une substance s'exprime par la diminution du pourcentage d'absorbance du radical libre testé.

Dans ces conditions de test, les extraits de Noix d'Alep sont plus actifs que les substances de référence telles que la vitamine E et la cynarine, qui sont les substances témoins testées les plus actives.

| Témoins | Concentration (mg/ml) | % Inhibition |
|---|---|---|
| Vitamine E | 0,01 | 9 |
| | 0,05 | 28 |
| | 0,1 | 61 |
| | 0.5 | 95 |
| | 1 | 96 |
| Cynarine | 0,01 | 16 |
| | 0,05 | 60 |
| | 0,1 | 89 |
| | 0,5 | 94 |
| | 1 | 96 |
| Extrait hydroéthanolique atomisé de Noix d'Alep | | |
| caractérisé en ce que la teneur en tanins par rapport au produit sec est de 80 % en poids et la teneur en acide gallique par rapport au produit sec est de 1,5 à 3 % en poids. | 0,01 | 15 |
| | 0,03 | 60 |
| | 0,05 | 73 |
| | 0,1 | 98 |
| | 0,3 | 100 |
| | 1 | 100 |

On a pu également évaluer l'activité anti-radicalaire des extraits obtenus selon l'invention sur l'anion superoxyde généré par la réaction du NADH (nicotinamide adénine dinucléotide hydrate) sur le PMS (phénazine méthosulfate), (Roback J., Gryglewski RJ, 1988, Biochemical Pharmacology, 37, (5), p. 837-841). L'anion superoxyde provoque la réduction du nitrobleu de tétrazolium en diformazan. De coloration bleue intense, celui-ci est dosé par spectrophotométrie. L'activité anti-radicalaire d'une substance est exprimée en pourcentage d'inhibition du développement de coloration due au diformazan. Par cette méthode, les extraits de Noix d'Alep provoquent une inhibition nettement plus marquée que des témoins comme l'acide rosmarinique ou la quercétine qui sont les substances témoins les plus actives dans ce test.

| Témoins | Concentration (mg/ml) | % Inhibition |
|---|---|---|
| ------- | | |
| Quercétine | 0,01 | 1 |
| | 0,05 | 31 |
| | 0,1 | 69 |
| | 0,5 | 91 |
| | 1 | 98 |
| Acide rosmarinique | 0,01 | 2 |
| | 0,05 | 28 |
| | 0,1 | 59 |
| | 0,5 | 90 |
| | 1 | 99 |
| Extrait hydroéthanolique atomisé de Noix d'Alep | | |
| ---------------------- | 0,01 | 19 |
| caractérisé en ce que | 0,02 | 47 |
| la teneur en tanins par | 0,03 | 71 |
| rapport au produit sec | 0,04 | 86 |
| est de 80 % en poids, et | 0,06 | 94 |
| la teneur en acide galli- | 0,08 | 98 |
| que par rapport au pro- | 0,1 | 100 |
| duit sec est de 1,5 à 3,0 % | 0,2 | 100 |
| en poids. | | |

Les extraits de Noix d'Alep de l'invention sont donc utilisables en cosmétique, en particulier pour la protection de la peau exposée à un ensoleillement prolongé, et pour retarder le processus radicalaire de vieillissement.

Les extraits de Noix d'Alep de l'invention peuvent être utilisés sous forme de pommades. Ces pommades sont préparées

- soit à partir d'un extrait de Noix d'Alep de l'invention, associé par exemple au mélange d'excipients suivant : monooléate de glycérol, vaseline, lanoline, cire blanche, huiles d'amande et de parrafine et eau,
- soit à partir d'un extrait de Noix d'Alep de l'invention, associé par exemple au mélange d'excipients suivant : huile d'arachide, lanoline anhydre, glycérine, amidon de blé, cire blanche et eau.

Les extraits de Noix d'Alep de l'invention peuvent également être présentés sous forme de crème dermique, lorsqu'ils sont associés à un mélange constitué d'excipients tels que la cire d'abeilles, la paraffine liquide légère, l'eau, la vaseline ou la lanoline, la cire blanche d'abeilles liquide légère et/ou le glycérol.

Enfin, des formules de produits solaires peuvent être préparées à partir des extraits de Noix d'Alep de l'invention : on peut incorporer l'extrait dans des émulsions classiques H/E (huile/eau) ou E/H (eau/huile) ou préparer simplement des solutions huileuses d'extrait.

EP 0 496 173 B1

**Revendications**

**Revendications pour les Etats contractants suivants : DE, GB, FR, IT, NL, SE, LI, CH, BE, AT, LU, DK**

1.  Extrait de Noix d'Alep, caractérisé par le fait qu'il contient de l'acide ellagique, de l'acide gallique et des tanins hydrolysables, et par le fait que la teneur en acide gallique est de 1,5 à 7 % en poids et que la teneur en tanins hydrolysables est de 65 à 85 % en poids par rapport à la poudre pour un extrait sec et par rapport au résidu sec pour un extrait liquide ou mou.

2.  Procédé d'obtention d'un extrait de Noix d'Alep selon la revendication 1, procédé caractérisé en ce que l'on extrait les Noix d'Alep à l'aide d'un solvant tel que l'eau, l'acétone, un alcanol en $C_1$-$C_4$, le propylèneglycol, ou un mélange de ces solvants.

3.  Procédé selon la revendication 2, caractérisé en ce que l'extraction est effectuée à l'aide d'un mélange alcanol en $C_1$-$C_4$/eau ou acétone/eau, mélange de 96/4 à 30/70 en volume.

4.  Procédé selon la revendication 2, caractérisé en ce que l'extraction est effectuée à l'aide d'un mélange propylèneglycol/eau, mélange de 100/10 à 40/60 en volume.

5.  Procédé selon l'une quelconque des revendications 2 à 4, caractérisé en ce que l'extraction est statique ou agitée.

6.  Procédé selon l'une quelconque des revendications 2 à 5, caractérisé en ce que la quantité de solvant utilisée égale 4 à 20 fois le poids de Noix d'Alep.

7.  Composition cosmétique, caractérisée en ce qu'elle contient un extrait de Noix d'Alep selon la revendication 1, en association avec tout excipient approprié.

8.  Composition cosmétique selon la revendication 7, caractérisée en ce qu'elle présente une activité anti-radicalaire.

9.  Composition cosmétique selon l'une quelconque des revendications 7 et 8, composition caractérisée en ce qu'elle présente une activité filtre U.V.B..

**Revendications pour les Etats contractants suivants : ES, GR**

1.  Procédé d'obtention d'un extrait de Noix d'Alep, caractérisé par le fait qu'il contient de l'acide ellagique, de l'acide gallique et des tanins hydrolysables, et par le fait que la teneur en acide gallique est de 1,5 à 7% en poids et que la teneur en tanins hydrolysables est de 65 à 85% en poids par rapport à la poudre pour un extrait sec et par rapport au résidu sec pour un extrait liquide ou mou, procédé caractérisé en ce que l'on extrait les Noix d'Alep à l'aide d'un solvant tel que l'eau, l'acétone, un alcanol en $C_1$-$C_4$, le propylèneglycol, ou un mélange de ces solvants.

2.  Procédé selon la revendication 1, caractérisé en ce que l'extraction est effectuée à l'aide d'un mélange alcanol en $C_1$-$C_4$/eau ou acétone/eau, mélange de 96/4 à 30/70 en volume.

3.  Procédé selon la revendication 1, caractérisé en ce que l'extraction est effectuée à l'aide d'un mélange propylèneglycol/eau 100/10 à 40/60 en volume.

4.  Procédé selon l'une quelconque des revendications 1 à 3, caractérisé en ce que l'extraction est statique ou agitée.

5.  Procédé selon l'une quelconque des revendications 1 à 4, caractérisé en ce que la quantité de solvant utilisée égale 4 à 20 fois le poids de Noix d'Alep.

7

**Patentansprüche**

**Patentansprüche für folgende Vertragsstaaten : DE, GB, FR, IT, NL, SE, LI, CH, BE, AT, LU, DK**

1. Extrakt von Aleppo-Galle, **dadurch gekennzeichnet**, daß er Ellagsäure, Gallussäure und hydrolysierbare Tannine enthält und daß sein Gehalt an Gallussäure 1,5 bis 7 Gew.-% und an hydrolysierbaren Tanninen 65 bis 85 Gew.-% beträgt, bezogen auf das Pulver im Fall eines trockenen Extrakts und auf den trockenen Rückstand im Fall eines flüssigen oder welchen Extrakts.

2. Verfahren zur Gewinnung eines Aleppo-Gallen-Extrakts nach Anspruch 1, **dadurch gekennzeichnet,** daß man Aleppo-Galle mit Hilfe eines Lösungsmittels, wie Wasser, Aceton, einem $C_1$-$C_4$-Alkanol, Propylenglykol oder einer Mischung dieser Lösungsmittel extrahiert.

3. Verfahren nach Anspruch 2, **dadurch gekennzeichnet,** daß die Extraktion mit einer $C_1$-$C_4$-Alkanol/Wasser- oder Aceton/Wasser-Mischung mit einem Volumenverhältnis von 96/4 bis 30/70 durchgeführt wird.

4. Verfahren nach Anspruch 2, **dadurch gekennzeichnet,** daß die Extraktion mit Hilfe einer Propylenglykol/Wasser-Mischung mit einem Volumenverhältnis von 100/10 bis 40/60 durchgeführt wird.

5. Verfahren nach einem der Ansprüche 2 bis 4, **dadurch gekennzeichnet,** daß die Extraktion statisch oder unter Bewegen erfolgt.

6. Verfahren nach einem der Ansprüche 2 bis 5, **dadurch gekennzeichnet,** daß die verwendete Lösungsmittelmenge dem 4- bis 20-fachen des Gewichts der Aleppo-Galle entspricht.

7. Kosmetische Zubereitung, **dadurch gekennzeichnet,** daß sie einen Aleppo-Gallen-Extrakt nach Anspruch 1 in Kombination mit einem geeigneten Trägermaterial enthält.

8. Kosmetische Zubereitung nach Anspruch 7, **dadurch gekennzeichnet,** daß sie eine gegen Radikale gerichtete Wirkung besitzt.

9. Kosmetische Zubereitung nach einem der Ansprüche 7 und 8, **dadurch gekennzeichnet,** daß sie eine UVB-Filterwirkung entfaltet.

**Patentansprüche für folgende Vertragsstaaten : ES, GR**

1. Verfahren zur Herstellung eines Extrakts von Aleppo-Galle, **dadurch gekennzeichnet,** daß er Ellagsäure, Gallussäure und hydrolysierbare Tannine enthält und daß sein Gehalt an Gallussäure 1,5 bis 7 Gew. -% und an hydrolysierbaren Tanninen 65 bis 85 Gew.-% beträgt, bezogen auf das Pulver im Fall eines trockenen Extrakts und auf den trockenen Rückstand im Fall eines flüssigen oder welchen Extrakts, **dadurch gekennzeichnet,** daß man Aleppo-Galle mit einem Lösungsmittel, wie Wasser, Aceton, einem $C_1$-$C_4$-Alkanol, Propylenglykol oder einer Mischung dieser Lösungsmittel extrahiert.

2. Verfahren nach Anspruch 1, **dadurch gekennzeichnet,** daß die Extraktion mit einer $C_1$-$C_4$-Alkanol/Wasser- oder Aceton/Wasser-Mischung mit einem Volumenverhältnis von 96/4 bis 30/70 durchgeführt wird.

3. Verfahren nach Anspruch 1, **dadurch gekennzeichnet,** daß die Extraktion mit Hilfe einer Propylenglykol/Wasser-Mischung mit einem Volumenverhältnis von 100/10 bis 40/60 durchgeführt wird.

4. Verfahren nach einem der Ansprüche 1 bis 3, **dadurch gekennzeichnet,** daß die Extraktion statisch oder unter Bewegen erfolgt.

5. Verfahren nach einem der Ansprüche 1 bis 4, **dadurch gekennzeichnet,** daß die verwendete Lösungsmittelmenge dem 4- bis 20-fachen des Gewichts der Aleppo-Galle entspricht.

## Claims

**Claims for the following Contracting States : DE, GB, FR, IT, NL, SE, LI, CH, BE, AT, LU, DK**

1. Gall apple extract, characterized in that it contains ellagic acid, gallic acid and hydrolysable tannins, and in that the gallic acid content is 1.5 to 7 % by weight and the hydrolysable tannin content is 65 to 85 % by weight relative to the powder in the case of a dry extract and relative to the solids content in the case of a liquid or soft extract.

2. Process for obtaining a gall apple extract according to Claim 1, characterized in that gall apples are subjected to extraction by means of a solvent such as water, acetone, a $C_1$-$C_4$ alkanol, propylene glycol or a mixture of these solvents.

3. Process according to Claim 2, characterized in that the extraction is carried out by means of a $C_1$-$C_4$ alkanol/water mixture or acetone/water mixture, in a ratio of 96/4 to 30/70 by volume.

4. Process according to Claim 2, characterized in that the extraction is carried out by means of a propylene glycol/water mixture in a ratio of 100/10 to 40/60 by volume.

5. Process according to any one of Claims 2 to 4, characterized in that the extraction is static or with stirring.

6. Process according to any one of Claims 2 to 5, characterized in that the amount of solvent used is equivalent to 4 to 20 times the weight of gall apples.

7. Cosmetic composition, characterized in that it contains a gall apple extract according to Claim 1 in combination with any suitable excipient.

8. Cosmetic composition according to Claim 7, characterized in that it has an antiradical activity.

9. Cosmetic composition according to any one of Claims 7 and 8, characterized in that it acts as a UV B filter.

**Claims for the following Contracting States : ES, GR**

1. Process for obtaining a gall apple extract characterized in that it contains ellagic acid, gallic acid and hydrolysable tannins, and in that the gallic acid content is 1.5 to 7 % by weight and the hydrolysable tannin content is 65 to 85 % by weight relative to the powder in the case of a dry extract and relative to the solids content in the case of a liquid or soft extract, this process being characterized in that the gall apples are subjected to extraction by means of a solvent such as water, acetone, a $C_1$-$C_4$ alkanol, propylene glycol or a mixture of these solvents.

2. Process according to Claim 1, characterized in that the extraction is carried out by means of a $C_1$-$C_4$ alkanol/water mixture or acetone/water mixture, in a ratio of 96/4 to 30/70 by volume.

3. Process according to Claim 1, characterized in that the extraction is carried out by means of a propylene glycol/water mixture in a ratio of 100/10 to 40/60 by volume.

4. Process according to any one of Claims 1 to 3, characterized in that the extraction is static or with stirring.

5. Process according to any one of Claims 1 to 4, characterized in that the amount of solvent used is equivalent to 4 to 20 times the weight of gall apples.